# EUROPEAN PATENT APPLICATION

(11) **EP 2 067 850 A1**
(43) Date of publication of application: **10.06.2009**
(21) Application number: 07822959.8
(22) Date of filing: 02.10.2007
(51) Int. Cl.: C12M 1/04, C12N 1/12

(54) **VERTICAL SUBMERSIBLE PHOTOBIOREACTOR FOR OBTAINING BIOFUELS**

(30) Priority: 02.10.2006 ES 200602507
(71) Applicant: Bio Fuel Systems, S.l., 03690 San Vicente de Raspeig (ES)
(72) Inventor: STROÏAZZO-MOUGIN, Bernard, A.J., E-03560 El Campello (Alicante) (ES); MENGUAL MOLINA, Rosa María, E-03002 Alicante (ES)
(74) Representative: Elzaburu Marquez, Alberto
(86) International application number: PCT/ES2007/000552
(87) International publication number: WO 2008/040828

(57) **Abstract**

The present invention relates to a vertical submersible photobioreactor for obtaining biofuels, consisting of: vertical towers (1) for performing photosynthesis, which can operate continuously using natural light inlets (12) and lamps (13) for producing artificial light, and a float system (17, 18) which can be used to submerge the towers (1) in the photic zone of aquatic environments, facilitating the thermal control thereof.

## Description

### Technical Field of the Invention

The present invention is comprised within the design of energy photobioconverters immersed in seas and lakes acting in a continuous and closed manner for producing biofuel and other products of interest by means of the mass culturing of autotrophic phytoplankton strains.

The invention is comprised within the technical field of the exploitation of renewable energy by means of the action of phytoplankton organisms that normally belong to the following taxonomic families: Chlorophyceae, Bacillariophyceae, Dinophyceae, Cryptophyceae, Chrysophyceae, Haptophyceae, Prasinophyceae, Raphidophyceae, Eustigmatophyceae ... generally the taxonomic families comprising species of the chromophyte division, all of them characterized by being flagellated or nonflagellated single-celled organisms and with a strictly planktonic (holoplanktonic) life phase, or at least one of its phases being planktonic (meroplanktonic).

Particularly by means of the use of energy photobioconverters, products such as biofuels, byproducts such as naphthas, kerosene, thermal energy, electric energy, free gases such as oxygen, hydrogen... are obtained.

It also promotes the massive uptake of greenhouse gases, especially carbon dioxide.

### State of the Art

Up until now, biofuels have been obtained from higher plant cultures, usually from the group of phanerogams or flowering plants (sunflowers, palm, European palm,..), and usually on the terrestrial surface (land plants).

The obligation for the economic zones to comply with the objectives imposed by the Kyoto protocol on the reduction of CO₂/SO₂ emissions and the emission of other gases causing the so-called greenhouse effect is forcing countries to search for alternative and renewable fuels to prevent possible penal taxes.

Although the production of solar and wind energy is increasing in some regions, these technologies are very expensive and are not viable in all climatic areas. In these conditions, biofuels have an important role as substitutes of fossil fuels, especially in transport and heating applications.

The production costs of biofuels from plants, such as palm and rapeseed oil have always been a reason for concern. Taking into account the low oil production indexes per hectare, enormous amounts of resources would be needed to reach commercial production. Land and water are two limited resources and it is preferable to use them to produce food products, which are furthermore more profitable for farmers. Intensive fertilization is furthermore a form of land and water pollution of the first order. Extensive single crop farming is also one of the main enemies of biodiversity.

Phytoplankton represents a viable solution to the previously discussed drawback given that about 50% of the dry mass of single-celled organisms is generally biofuel. In addition, the annual production per hectare of biofuel from phytoplankton is 40 times higher than with the second most cost-effective product, palm oil. A drawback is that the production of phytoplankton oil requires covering vast stretches of land with rather shallow water, as well as introducing large amounts of CO₂, an essential element for phytoplankton to produce oil. Natural production systems such as phytoplankton ponds, have a relatively low cost but the harvesting process is very laborious and, therefore expensive. On the other hand, phytoplankton culturing is carried out in open systems, making it vulnerable to contamination and to problems for cultures, which may lead to total production loss. In this same sense, an advantage of the photoconverter described in the present invention is that the system is kept closed and in conditions such that the culture is not contaminated by bacteria, fungi,... because in addition to being closed, the culture is enriched by means of nutrients incorporating fungicides and antibiotics.

Within the field of the design of photobioconverters for producing biofuels through photosynthetic microorganisms, two types of photobioconverters could be clearly differentiated: open photobioconverters, in which a direct exchange of matter between the culture and the air surrounding it is allowed, and closed photobioconverters, in which this exchange is eliminated by means of the placement of a transparent physical medium allowing the passage of electromagnetic radiation but not the exchange of matter. The open photobioconverters have many problems derived from the little control of the culturing conditions and possible contaminations, so their application is limited due to these drawbacks. However, closed photobioconverters efficiently reduce these problems by means of greater control of the culturing conditions and possible contaminations and can reach a production rate that is 400 times higher than the production rate of sunflower.

Until now no systems similar to the photobioconverter object of the present invention have been described which incorporate the advantages of being a closed system with a large volume and large diameters, which works continuously, which allows obtaining large amounts of biofuels or byproducts such as naphthas, glycerin, silicon-derived compounds, such as ferrosilicates, which may further obtain thermal and electric energy and does not contaminate given that all the possible residues, such as carbon dioxide, are recirculated in the system to be used as a nutrient for the phytoplankton, or which recirculates the water used as part of the culture medium so it can be reused.

The present invention also describes and is clearly differentiated from any other type of photobioconverter in its capacity to be arranged or located immersed in liquid medium (oceans, seas, lakes, reservoirs, ...). An essential feature of the invention differentiating it with respect to those developed in land medium is that due to its immersed arrangement, it facilitates the thermal regulation of the system, which in turn facilitates the control of the phytoplankton populations which are being cultured and decreases the necessary energy costs to maintain the homeothermal conditions in the culturing system. As a second feature, it ensures water availability with no type of limitation and high expenses in infrastructures (unlike what occurs on land).

As a third differentiating feature with respect to that already described, the present invention allows immersing the photobioconverters inside the entire photic zone, which allows controlling, without energy expenditure, the partial pressure of the gases involved in the process, thus facilitating the process for absorbing or eliminating said gases as appropriate.

The present invention therefore describes a novel system (photobioconverter) including all these features which allows enormous versatility and is very environmentally friendly.

Patent application WO 03/094598 A1 entitled "Photobioreactor and process for biomass production and mitigation of pollutants in flue gases" describes a generic photobioreactor model mainly focused on decontaminating COx, SOx and NOx type gases. It is basically a system working in a discontinuous manner (distinguishing between day/night photoperiod) and is open, its liquid medium not being axenic. It does not control nitrogen and carbon dioxide concentrations for the purpose of increasing biofuel production. It is not designed to work with monospecific or monoclonal algae strains. Its design does not contemplate biofuel production as the main objective, rather it is focused on gas purification. On the other hand, as regards the photosynthetic organisms it refers to, it does not demand conditions disabling the system and it has no controlled recirculation because the transport is done by a turbulent flow of bubbles.

Compared to the present invention object of the patent, a completely novel system is set forth which is based, in contrast, on the following features:
- It is mainly designed for an aquatic medium although its use is not restricted to the terrestrial surface if small modifications are applied to it.
- It is completely closed.
- It is completely axenic.
- It works continuously without distinguishing photoperiods.
- It works with monospecific and monoclonal strains.
- It accepts mixed autotrophic-autotrophic, autotrophic-heterotrophic, facultative heterotrophic-facultative heterotrophic cultures.
- It does not accept any photosynthetic organism, but rather it at least requires that they are not organisms forming biofouling on the inner surface of the photobioconverter.
- It accepts facultative heterotrophic organisms.
- It requires that the phytoplankton species do not form colonies.
- It requires that the phytoplankton species do not generate exo-mucilage.
- It requires that the cultured species contains at least 5% of fatty acids and at least 5% of hydrocarbons.
- It enhances the use of nonflagellated and floating phytoplankton species.
- It does not accept any type of liquids as culture medium, it focuses on freshwater, brackish water and sea water.
- Its main objective is focused on obtaining metabolic synthesis compounds with energetic properties or with pre-energetic properties essentially aimed at obtaining biofuels.

### Description

The present invention relates to an energy photobioconverter for obtaining biofuels including, but not limited to, biopetroleum, for fixing carbon dioxide and greenhouse gases and other, though not less important, byproducts.

Biopetroleum is understood as an energetic liquid produced by means of converting electromagnetic energy into chemical energy through the phytoplankton biomass which has the same origin as the fossil fuel, petroleum, but in the present invention the same energetic product has been extracted without it fossilizing.

Said energy photobioconverter can be immersed in a liquid medium and, in a non-limiting sense, in seas and lakes (they are connected to the shore by feed-extraction tubes for the energetic products), acting in a continuous and closed manner for producing biofuel and other products of interest by means of the mass culturing of autotrophic phytoplankton strains.

The water line is determined according to the culture medium and parameters such as light, pressure, temperature and natural convection flow of the environment.

In addition, the photobioconverters of the present invention use a Tichelmann-type flow control system which allows providing equal pressure in any part thereof and thus continuously controls the extraction.

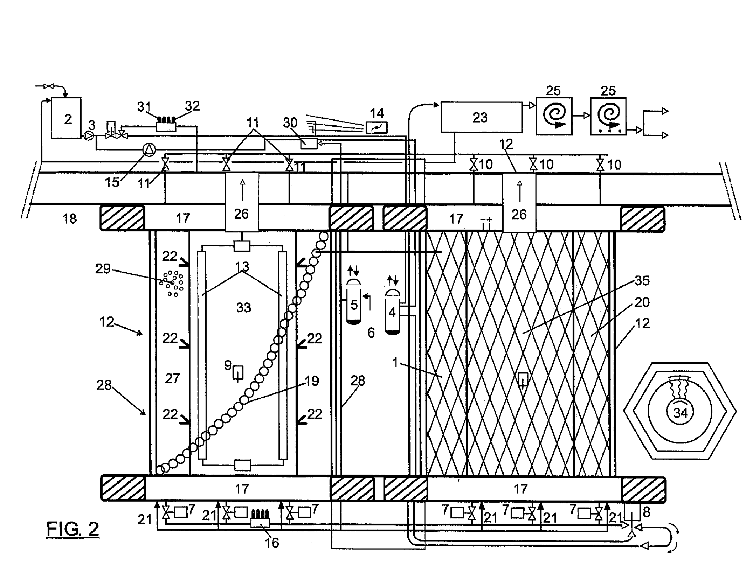

A first aspect of the present invention consists of a photobioconverter formed by at least the following elements, as shown in Figures 2, 3 and 4:
- at least 1 photosynthesis conducting tower (1) for each photobioconverter module which can be of three types: circular single-chamber, circular concentric two-chamber and composite circular containing vertical tubes arranged around a central light well.
- at least 2 mixture and buffer tanks (2) for each photobioconverter module.
- at least 2 reinjection and pressure control pumps (3) for each photobioconverter module.
- at least 2 heat exchangers (4) to maintain the photobioconverter temperature for each photobioconverter module.
- at least 1 desuperheater (5) to reduce the inlet temperature of carbon dioxide (), hereinafter CO₂, for each photobioconverter module.
- at least 6 electromagnetic flow control valves (7) for each photobioconverter module.
- at least 6 electromagnetic extraction valves (8) for each photobioconverter module.
- at least 3 control sensors (9) of the culture medium for each photobioconverter module.
- at least 3 oxygen extraction valves (10) for each photobioconverter module.
- at least 3 hydrogen extraction valves (11) for each photobioconverter module.
- 100% of natural light inlets (12) of the useful surface.
- at least 30 artificial lighting lamps (13) for each photobioconverter module.
- at least 1 control panel (14) for each photobioconverter module.
- at least 1 recirculation pump (15) for each photobioconverter module.
- at least 2 densimeters (16) for each photobioconverter module.
- at least 1 reinjection and pressure control pump (3) of the liquid from (23) and for each photobioconverter module.
- at least 1 decanter (23) for each photobioconverter module.
- at least 1 flotation and accumulation tank (17) for each photobioconverter module.
- at least 2 floats (18) for each photobioconverter module for each photobioconverter module.
- at least 1 rotating cleaning system (19) for each photobioconverter module.
- at least 1 cleaning and anti-external fouling organism system (20) for each photobioconverter module.
- at least 15 carbon dioxide injection valves (21) arranged in a helical manner around the photosynthesis conducting towers (1) for each photobioconverter module.
- at least 15 turbulence injection valves (22) arranged in a helical manner for each photobioconverter module.
- at least 1 artificial lighting lamp extraction and regulation system (24) for each photobioconverter module.
- at least 1 mechanical biomass extraction system (25) by centrifugation for each photobioconverter module.
- at least 1 electromagnetic molecular exchange accelerating system (37) for each photobioconverter module.

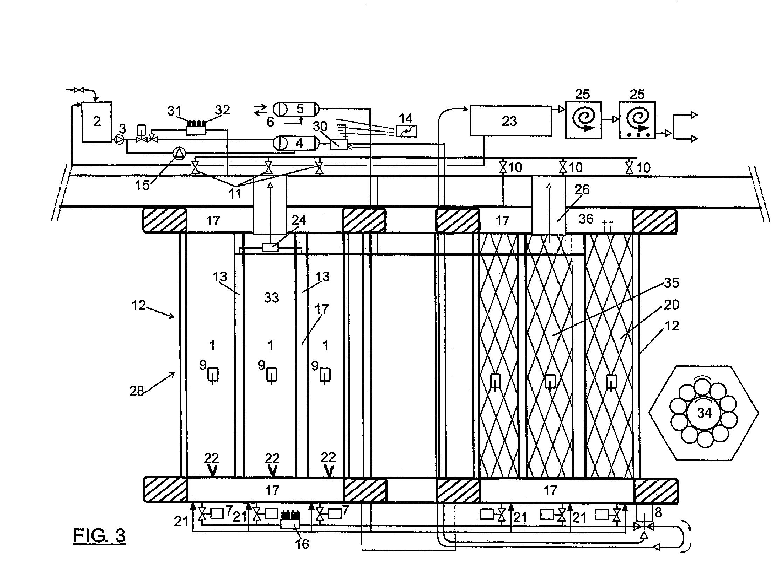

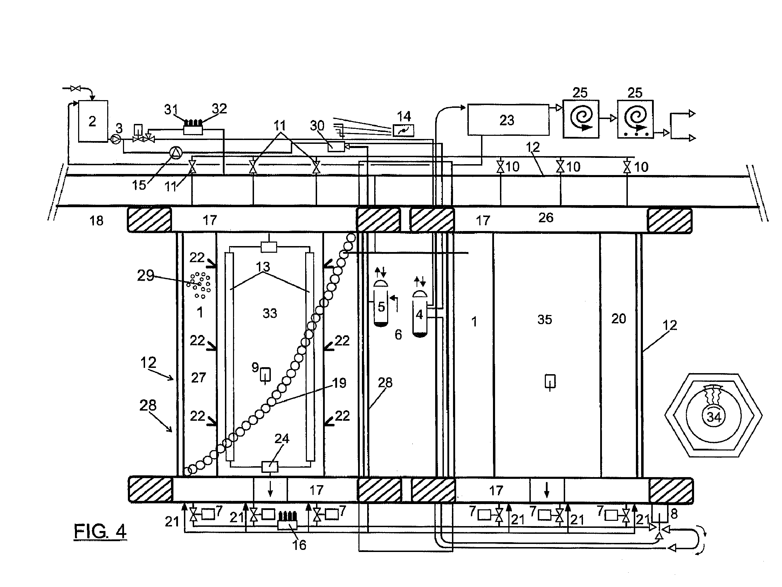

The photosynthesis conducting towers (1) are made of a transparent material, preferably PVC, polycarbonate and/or methacrylate and can be of three different types:
- circular concentric single-chamber (Figure 2).
- circular concentric two-chamber (Figure 4).
- composite circular containing vertical tubes arranged around a central light well (Figure 3).

And these three types of photosynthesis conducting towers (1) are furthermore the space from the outer face of the light well to the inner face of the photosynthesis tower, ranging from 10 centimeters a 1 meter and with a height from 10 to 30 meters.

In this same sense, circular concentric single-chamber photosynthesis conducting towers (1) comprise the following elements:
- accessible vertical wells for the control, maintenance and emission of artificial light (26), having a diameter comprised from 20 centimeters to 2 meters and a height comprised from 10 to 30 meters.
- photosynthesis chambers (27).

The circular concentric two-chamber photosynthesis conducting towers (1) comprise the following elements:
- accessible vertical wells for the control, maintenance and emission of artificial light (26).
- photosynthesis chambers (27).
- external heat stabilization chambers (28).

The photosynthesis conducting towers (1) comprise at least the following elements:
- densimeters (15).
- CO₂ injection valves (21).
- turbulence injection valves (22).
- flow control valves (7).
- natural light inlets (12).
- artificial lighting lamps (13).
- recirculation pumps (15).
- control panels (14).
- phytoplankton (29).
- internal illumination systems (33).

The photosynthesis conducting towers (1) can additionally contain electromagnets (34) in the exterior to accelerate the molecular electron exchange (37).

The mixture and buffer tanks (2) are cylindrical or polyhedral, made of a transparent material, preferably PVC, polycarbonate and/or methacrylate; they have an internal volume comprised within the range of 3 to 14m³ per photobioconverter and allow the assembly between the different photosynthesis towers (1) in a manner similar to a beehive structure. In the same sense, the mixture and buffer tanks (2) contain the mixture of necessary nutrients and gases for the development and culturing of the phytoplankton.

The reinjection and pressure control pumps (3) are centrifugal-type pumps and have a flow comprised within the range of 4 to 100 cm/sec.

The heat exchangers (4) are useful for maintaining the temperature of the system, and the desuperheaters (5), having the function of reducing the inlet temperature of CO₂ and NO_{x'}, are laminar flow plate-type desuperheaters.

The photobioconverter additionally contains ion sprayers (30) ionizing the nutrients and a better and more efficient assimilation of such nutrients by the phytoplankton, gas sensors (31) and photosensors (32) is thus allowed.

An ion sprayer (30) is understood as any system known in the state of the art that is able to ionize molecules.

The control sensors (9) control the temperature, pH, salinity, conductivity, CO₂, O₂, trace element, antibiotic and fungicide concentration.

The biomass containing lipids, carbohydrates, celluloses, hemicelluloses and products from secondary metabolism is separated in the mechanical extraction systems (25) by centrifugation.

The natural light inlets (12) are coated with translucent plastic.

The control panels (14) control the injection of the different nutrients, gases, temperature, pH, salinity and conductivity of the culture medium.

The flotation and accumulation tanks (17) are translucent and have a lattice stainless steel structure and comprise at least the following elements:
- photosensors (32).
- CO₂ and air valves (21).
- electromagnetic or pneumatic extraction valves (8).
- floats (18).

The rotating cleaning systems (19) are in the form of balls joined by a central thread which, by means of a centrifugal, helical rotating movement system, runs along the inner walls of the photobioconverter, keeping it clean.

The cleaning and anti-fouling organism systems (20) surround all the parts in contact with the water outside the photobioconverter and comprise the following elements:
- copper wire mesh (35) with a gage of 0.1 to 0.2 millimeters and a mesh size of 4 cm.
- contact electrodes (36) for the mesh arranged in the flotation tanks (17) and mixture and buffer tanks (2).

The culturing conditions for the phytoplankton present in the photosynthesis conducting towers (1) inside the photobioconverter are as follows:
- temperature from 12 to 35 degrees Celsius.
- sunlight intensity from 200 to 900 watts/m².
- artificial light intensity from 1 to 50 watts/m².
- photoperiods from 6 p.m. to 6 a.m. or from 12 to 12 or of 24 hours.
- salinity from 0 per thousand up to 50 per thousand.
- phytoplankton concentration in the culture medium from 1,000,000 cells/ml to 100,000,000 cells/ml.
- pH from 7 to 8.9.

According to a second essential aspect of the present invention, the use of the photobioconverter is for obtaining biofuels, for obtaining pharmacopoeia products such as lutein and fatty acids, for obtaining cosmetic products such as emulsifying substances, pigments and glycerin, for obtaining industrial products with a silica content such as borosilicates and ferrosilicates, for obtaining fertilizer, agricultural, industrial and livestock products, for obtaining celluloses and hemicelluloses, for obtaining tannins and astringent compounds, for fixing CO₂, CH₄, SH₂, NO₂, NO₃ and other greenhouse gases.

The antibiotics added to the culture are a mixture of penicillin and streptomycin at a concentration range from 100 to 300 mg/l each, preferably at a concentration range from 150 to 250 mg/l and more preferably at a concentration of 200 mg/l for each of the components of the mixture.

The fungicides added to the culture are a mixture of griseofulvin and nystatin at a concentration range from 100 to 300 mg/l each, preferably at a concentration range from 150 to 250 mg/l and more preferably at a concentration of 200 mg/l for each of the components of the mixture.

The water added for the phytoplankton culture can be freshwater, brackish water or salt water.

The electromagnetic flow control or extraction valves (7) are located at the base of the photosynthesis conducting towers (1) and depend on photovalves operating by the difference of the light intensity between two points (controlled opening).

The decanters (23) separate the biomass produced by the phytoplankton from the water of the culture medium. These decanters are static decanters. The separated biomass contains, among other products and in no limiting sense, lipids, carbohydrates and products from the secondary metabolism of the phytoplankton.

The recirculation pumps (15) cause a Venturi-type effect which consists of the pressure of the fluid in the current of a fluid inside a closed conduit being reduced as the velocity increases when it passes through an area having a smaller section. If at this point of the conduit the end of another conduit is introduced, the fluid contained in this second conduit is suctioned for recirculation to prevent the algae from being destroyed due to the pressure.

### Embodiment

An inoculum of a phytoplankton strain *(Nannochloropsis gaditana*) is introduced in (2), culture medium, nutrients, CO₂, air and nutrients are added which are detected and regulated by means of (31) and (32) in the same way in (2). Circulation thus begins so as to establish a continuous flow within which the phytoplankton cells will travel, at the same time reproducing through (1) where they are insufflated with carbon dioxide (6a) coming from (6) which controls the temperature and are ionized in (30), and then they will move to (1), uptaking the electromagnetic energy in order to conduct photosynthesis. This is where the luminous intensity is controlled by means of (light well) and it is in (34) where the electromagnetic field aids in polarizing the CO₂ molecules, thus aiding in their dissolution and thus facilitating the uptake thereof and increasing the biomass produced, rich in lipids, carbohydrates, celluloses and hemicelluloses, among other products. The process is repeated in each photosynthesis tower. Inside each photosynthesis tower, O₂ is released and lost by means of (11) and is detected by (12), and the same occurs for H₂ (12) and is detected by (12). A recirculation process (15) is carried out within a Tichelmann-type pressure equilibrium process until the phytoplankton biomass is sufficient and detected by (16) in order to begin the extraction of part of it through (8) and from there the extracted part goes to (23), in which the water is separated from the biomass and is again propelled to the recirculation systems, finally going to (2).

The initial inoculum of the producing phytoplankton strain is carried out such that the initial volume contained in each photosynthesis tower starts with a cell concentration of at least 1,000,000 cells/ml. Between 6 and 8 days later, the cell concentration in the photosynthesis towers will range from 100 to 200 million cells/ml. Once this time is reached, biomass extractions will be periodically undertaken according to the concentration existing at each time.

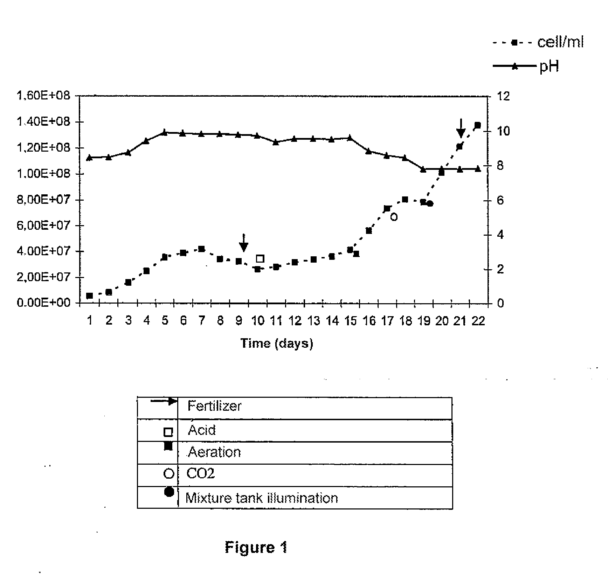

All this data is shown in Table 1 and the results are shown in Figure 1.

**Table 1**

| Time (days) | Cells/ml | pH | Fertilizer | Acid | Aeration | CO₂ | Light |
|---|---|---|---|---|---|---|---|
| 1 | 5.57 x 10⁶ | 8.45 | | | | | |
| 2 | 8.39 x 10⁶ | 8.47 | | | | | |
| 3 | 16.2 x 10⁶ | 8.74 | | | | | |
| 4 | 25.1 x 10⁶ | 9.43 | | | | | |
| 5 | 35.7 x 10⁶ | 9.91 | | | | | |
| 6 | 38.9 x 10⁶ | 9.87 | | | | | |
| 7 | 42.2 x 10⁶ | 9.83 | | | | | |
| 8 | 34.5 x 10⁶ | 9.82 | | | | | |
| 9 | 32.7 x 10⁶ | 9.79 | 40 ml | | | | |
| 10 | 26.7 x 10⁶ | 9.74 | | 300 ml | | | |
| 11 | 28.4 x 10⁶ | 9.36 | | | | | |
| 12 | 32.1 x 10⁶ | 9.56 | | | | | |
| 13 | 34.1 x 10⁶ | 9.57 | | | | | |
| 14 | 36.6 x 10⁶ | 9.54 | | | | | |
| 15 | 41.5 x 10⁶ | 9.62 | - | | increases | | |
| 16 | 56.6 x 10⁶ | 8.86 | | | | | |
| 17 | 73.9 x 10⁶ | 8.61 | | | | Connected | |
| 18 | 81.0 x 10⁶ | 8.46 | | | | | |
| 19 | 78.9 x 10⁶ | 7.82 | | | | | increases |
| 20 | 102 x 10⁶ | 7.84 | | | | | |
| 21 | 122 x 10⁶ | 7.82 | 40 ml | | | | |
| 22 | 138 x 10⁶ | 7.84 | | | | | |

The pH will be one of the main indicators of the productive stability of the system. It must be maintained using the introduced CO₂ flow rate, the introduced air flow rate and by means of the calcium carbonate-rich inert material beds, which will be arranged inside the mixture and buffer tanks. The pH will range from 7.0 - 8.9

The necessary light radiation will range between 15.0 MJ.m⁻².d⁻¹ and 1.0 MJ.m-².d⁻¹. It will be provided through the light well (33).

### Brief Description of the Drawings

Figure 1 shows a representative diagram of the evolution of the culture in the photobioconverter object of the present invention with each of its parts and fittings for the use of artificial and solar electromagnetic energy for the purpose of obtaining, among other products, biofuel, celluloses, hemicelluloses and for reducing greenhouse gases in the atmosphere, especially CO₂, CH₄, NOₓ and SOₓ.
Figure 2 shows a diagram of the circular concentric single-chamber-type energy photobioconverter immersed in a liquid medium. Said type of energy photobioconverter is immersed in seas and lakes, connected to the shore by feed-extraction tubes for the energetic products and acts in a continuous and closed manner for producing biofuel and other products of interest by means of the mass culturing of autotrophic phytoplankton strains.
Figure 3 shows a diagram of the composite circular-type energy photobioconverter immersed in a liquid medium, containing vertical tubes arranged around a central light well. Said type of energy photobioconverter is immersed in seas and lakes, connected to shore by feed-extraction tubes for the energetic products and acts in a continuous and closed manner for producing biofuel and other products of interest by means of the mass culturing of autotrophic phytoplankton strains.
Figure 4 shows a diagram of the circular concentric two-chamber-type energy photobioconverter on land, in a coastal area. Said energy photobioconverter acts in a continuous and closed manner for producing biofuel and other products of interest by means of the mass culturing of autotrophic phytoplankton strains. It is placed on pillars outside the liquid medium according to light and temperature gradients.

## Claims

1. An energy photobioconverter for obtaining biofuels, **characterized in that** it comprises at least the following elements:
a. photosynthesis conducting towers (1);
b. mixture and buffer tanks (2);
c. reinjection and pressure control pumps (3);
d. heat exchangers (4) to maintain the photobioconverter temperature;
e. desuperheaters (5) to reduce the inlet temperature of CO₂ (6);
f. electromagnetic flow control valves (7);
g. electromagnetic extraction valves (8);
h. control sensors (9) of the culture medium;
i. oxygen extraction valves (10);
j. hydrogen extraction valves (11);
k. natural light inlets (12);
l. artificial lighting lamps (13);
m. control panels (14);
n. recirculation pumps (15);
o. densimeters (16);
p. flotation and accumulation tanks (17);
q. floats (18);
r. rotating cleaning systems (19);
s. cleaning and anti-external fouling organism systems (20);
t. CO₂ injection valves (21);
u. turbulence injection valves (22);
v. decanters (23)
w. artificial lighting lamp extraction and regulation systems (24);
x. mechanical extraction systems (25) by centrifugation; and
y.- electromagnetic molecular exchange accelerating system (37).

2. An energy photobioconverter for obtaining biofuels according to claim 1, **characterized in that** the photosynthesis conducting towers (1) are circular concentric single-chamber-type towers.

3. An energy photobioconverter for obtaining biofuels according to claim 1, **characterized in that** the photosynthesis conducting towers (1) are circular concentric two-chamber-type towers.

4. An energy photobioconverter for obtaining biofuels according to claim 1,
**characterized in that** the photosynthesis conducting towers (1) are composite circular-type towers containing vertical tubes arranged around a central light well.

5. An energy photobioconverter for obtaining biofuels according to claim 2, **characterized in that** the circular concentric single-chamber photosynthesis conducting towers (1) comprise the following elements:
a. accessible vertical wells for the control, maintenance and emission of artificial light (26); and
b. photosynthesis chambers (27).

6. An energy photobioconverter for obtaining biofuels according to claim 5, **characterized in that** the vertical wells (26) have a diameter comprised from 20 centimeters to 2 meters and a height comprised from 10 to 30 meters.

7. An energy photobioconverter for obtaining biofuels according to claim 3, **characterized in that** the circular concentric two-chamber photosynthesis conducting towers (1) comprise the following elements:
a. accessible vertical wells for the control, maintenance and emission of artificial light (26);
b. photosynthesis chambers (27); and
c. external heat stabilization chambers (28).

8. An energy photobioconverter for obtaining biofuels according to claim 1, **characterized in that** the photosynthesis conducting towers (1) comprise at least the following elements:
a. densimeters (15);
b. CO₂ injection valves (21);
c. turbulence injection valves (22);
d. flow control valves (7);
e. natural light inlets (12);
f. artificial lighting lamps (13);
g. recirculation pumps (15);
h. control panels (14); and
i. phytoplankton (29).

9. An energy photobioconverter for obtaining biofuels according to claim 1, **characterized in that** the mixture and buffer tanks (2) are cylindrical or polyhedral.

10. An energy photobioconverter for obtaining biofuels according to claims 1 and 9, **characterized in that** the mixture and buffer tanks (2) allow the assembly between different photosynthesis towers in a manner similar to a beehive structure.

11. An energy photobioconverter for obtaining biofuels according to claims 1 and 9 to 10, **characterized in that** the mixture and buffer tanks (2) contain the mixture of necessary nutrients and gases for the development and culturing of the phytoplankton.

12. An energy photobioconverter for obtaining biofuels according to claims 1 and 9 to 11, **characterized in that** the mixture and buffer tanks (2) are made of a transparent material, preferably PVC, polycarbonate and/or methacrylate.

13. An energy photobioconverter for obtaining biofuels according to claims 1 and 9 to 12, **characterized in that** the mixture and buffer tanks (2) have an internal volume comprised within the range of 3 to 14m³ per photobioconverter.

14. An energy photobioconverter for obtaining biofuels according to claim 1, **characterized in that** the recirculation pumps (15) have a flow comprised within the range of 4 to 100 cm/sec.

15. An energy photobioconverter for obtaining biofuels according to claims 1 and 14, **characterized in that** the recirculation pumps (15) are centrifugal-type pumps.

16. An energy photobioconverter for obtaining biofuels according to claim 1, **characterized in that** the heat exchangers (4) and the desuperheaters (5) are laminar flow plate-type heat exchangers and desuperheaters.

17. An energy photobioconverter for obtaining biofuels according to claim 1, **characterized in that** it additionally contains ion sprayers (30).

18. An energy photobioconverter for obtaining biofuels according to the previous claim, **characterized in that** the ion sprayers (30) ionize the nutrients.

19. An energy photobioconverter for obtaining biofuels according to claim 1, **characterized in that** the control sensors (9) control the temperature, pH, salinity, conductivity, CO₂, O₂, trace element, antibiotic and fungicide concentration.

20. An energy photobioconverter for obtaining biofuels according to claim 1, **characterized in that** it additionally contains gas sensors (31).

21. An energy photobioconverter for obtaining biofuels according to claim 1, **characterized in that** it additionally contains photosensors (32).

22. An energy photobioconverter for obtaining biofuels according to claim 1, **characterized in that** the biomass containing lipids, carbohydrates, celluloses, hemicelluloses and products from secondary metabolism is separated in the mechanical extraction systems (25) by centrifugation.

23. An energy photobioconverter for obtaining biofuels according to claim 1, **characterized in that** the natural light inlets (12) are coated by translucent plastic.

24. An energy photobioconverter for obtaining biofuels according to claim 1,
**characterized in that** the photosynthesis conducting towers (1) contain internal illumination systems (33).

25. An energy photobioconverter for obtaining biofuels according to claim 1, **characterized in that** the photosynthesis conducting towers (1) are made of a transparent material, preferably PVC, polycarbonate and/or methacrylate.

26. An energy photobioconverter for obtaining biofuels according to claim 1, **characterized in that** the photosynthesis conducting towers (1) additionally contain electromagnets (34) in the exterior to accelerate the molecular electron exchange.

27. An energy photobioconverter for obtaining biofuels according to claim 1, **characterized in that** the control panels (14) control the injection of the different nutrients, gases, temperature, pH, salinity and conductivity of the culture medium.

28. An energy photobioconverter for obtaining biofuels according to claim 1, **characterized in that** the flotation and accumulation tanks (17) are translucent and have a lattice stainless steel structure.

29. An energy photobioconverter for obtaining biofuels according to claims 1 and 28, **characterized in that** the flotation and accumulation tanks (17) comprise at least the following elements:
a. photosensors (32);
b. CO₂ and air valves (21);
c. electromagnetic or pneumatic extraction valves (8); and
d. floats (18).

30. An energy photobioconverter for obtaining biofuels according to claim 1, **characterized in that** the rotating cleaning systems (19) is in the form of balls joined by a central thread which, by means of a centrifugal, helical rotating movement system, runs along the inner walls of the photobioconverter, keeping it clean.

31. An energy photobioconverter for obtaining biofuels according to claim 1, **characterized in that** the cleaning and anti-fouling organism systems (20) surround all the parts in contact with the water outside the photobioconverter.

32. An energy photobioconverter for obtaining biofuels according to claims 1 and 31, **characterized in that** the cleaning and anti-fouling organism systems (20) comprise the following elements:
a. copper wire mesh (35) with a gage of 0.1 to 0.2 millimeters and a mesh size of 4 cm; and
b. contact electrodes (36) for the mesh arranged in the flotation tanks (17) and mixture and buffer tanks (2).

33. An energy photobioconverter for obtaining biofuels according to any of the previous claims, **characterized in that** it comprises the following culturing conditions for the phytoplankton present in the photosynthesis conducting towers (1):
a. temperature from 12 to 35 degrees Celsius;
b. sunlight intensity from 200 to 900 watts/m²;
c. artificial light intensity from 1 to 50 watts/m²;
d. photoperiods from 6 p.m. to 6 a.m. or from 12 to 12 or of 24 hours;
e. salinity from 0 per thousand up to 50 per thousand;
f. phytoplankton concentration in the culture medium from 1,000,000 cells/ml to 100,000,000 cells/ml; and
g. pH from 7 to 8.9.

34. The use of the energy photobioconverter according to any of the previous claims for obtaining biofuels.

35. The use of the energy photobioconverter according to any of the previous claims for obtaining pharmacopoeia products such as lutein and fatty acids.

36. The use of the energy photobioconverter according to any of the previous claims for obtaining cosmetic products such as emulsifying substances, pigments and glycerin.

37. The use of the energy photobioconverter according to any of the previous claims for obtaining industrial products with a silica content such as borosilicates and ferrosilicates.

38. The use of the energy photobioconverter according to any of the previous claims for obtaining fertilizer, agricultural, industrial and livestock products.

39. The use of the energy photobioconverter according to any of the previous claims for obtaining celluloses and hemicelluloses.

40. The use of the energy photobioconverter according to any of the previous claims for obtaining tannins and astringent compounds.

41. The use of the energy photobioconverter according to any of the previous claims for fixing CO₂, CH₄, SH₂, NO₂, NO₃ and other greenhouse gases.
